# EUROPEAN PATENT SPECIFICATION

(11) **EP 1 814 621 B1**
(45) Date of publication and mention of the grant of the patent: **31.03.2010**
(21) Application number: 05820457.9
(22) Date of filing: 24.11.2005
(51) Int. Cl.: A61M 16/22, A62B 19/00

(54) **IMPROVEMENTS RELATING TO RESPIRATORY CIRCUITS**
VERBESSERUNGEN VON ATEMWEGSKREISLÄUFEN
AMELIORATIONS CONCERNANT DES CIRCUITS RESPIRATOIRES

(30) Priority: 24.11.2004 GB 0425782
(43) Date of publication of application: 08.08.2007
(73) Proprietor: Intersurgical AG, Vaduz (LI)
(72) Inventor: Pittaway, Alan, Buckinghamshire HP13 5QP (GB); Holder, Michael John, Ox fordshire OX9 5LX (GB)
(74) Representative: Adamson Jones
(86) International application number: PCT/GB2005/004777
(87) International publication number: WO 2006/056807

(56) References cited:
- WO-A-03/092817
- CH-A- 207 614
- CH-A- 215 781
- DE-C- 891 052
- FR-A- 881 620
- US-A- 3 752 654

## Description

This invention relates to respiratory circuits, and in particular to respiratory circuits including cartridges of material for treating respiratory gases.

In anaesthetic respiratory circuits, chemical absorbents are generally used to remove carbon dioxide from exhaled respiratory gases. In such respiratory circuits, the chemical absorbent is usually contained within a cartridge incorporated into the respiratory circuit. In particular, such cartridges include an inlet at one end of the cartridge and an outlet at the other end of the cartridge such that exhaled respiratory gases flow through the interior of the cartridge and are treated by the chemical absorbent contained therewithin.

A variety of different forms of such chemical absorbents are well known in the art (see e.g. WO 03/092817 or US 3752654), but the chemical absorbent is usually granular in form. The inlet and outlet conventionally take the form of circular meshes located at opposite ends of a generally cylindrical cartridge. The chemical absorbent normally includes a pH indicator, such as ethyl violet, that changes colour, eg from colourless to violet in the case of ethyl violet, when the chemical absorbent is exhausted and hence no longer able to effectively absorb carbon dioxide. At least a side wall of the cartridge is therefore usually sufficiently translucent for such a colour change to be visible to a user. Most chemical absorbents also produce heat and water on reaction with the respiratory gases, and so act to humidify and heat the respiratory gases flowing through the cartridge.

The circular mesh that forms the inlet is generally of lesser diameter than the diameter of the cartridge. For this reason, conventional cartridges generally suffer from the major disadvantage that respiratory gases tend to flow predominantly through a central cylindrical channel of the interior of the cartridge and hence a central cylindrical core of the chemical absorbent.

As a consequence, a central cylindrical core of the chemical absorbent typically becomes exhausted before the surrounding chemical absorbent, and hence there is non-uniform use of the chemical absorbent within the cartridge.
The cartridge will therefore become ineffective and will need to be replaced before an outer portion of the chemical absorbent has been fully exhausted. This is clearly a waste of chemical absorbent. In addition, any colour change of the chemical absorbent in the central core will not be visible through the side wall of the container, and hence a user is given no indication of the inactivity of the cartridge until a carbon dioxide warning indicator, which is conventionally included in a respiratory circuit, is activated.

There has now been devised an improved cartridge for incorporation within a respiratory circuit which overcomes or substantially mitigates the above-mentioned and/or other disadvantages associated with the prior art.

According to a first aspect of the invention, there is provided a cartridge for incorporation within a respiratory circuit, the cartridge being adapted to contain a material for treating respiratory gases and comprising an inlet and an outlet such that respiratory gases flow, in use, through the interior of the cartridge and interact with said material, characterised in that the inlet comprises a plurality of openings arranged in an annulus about a continuous central part of a base of the cartridge, and said openings are in communication with a plurality of channels on an interior surface of the cartridge for guiding the respiratory gas transversely relative to its direction of flow through the interior of the cartridge.

The cartridge according to the invention is advantageous principally because the respiratory gas can be guided transversely so as to flow substantially uniformly through the interior of the cartridge. The present invention therefore significantly reduces the amount of material for treating the respiratory gases that is wasted by ensuring that the material is uniformly active throughout the interior of the cartridge during use. This increases the lifetime of a given size of cartridge, and hence reduces cost.

The cartridge according to the invention is suitable for incorporation within a respiratory circuit, such as an anaesthetic respiratory circuit. Typically, the material for treating respiratory gases will be a chemical absorbent for absorbing, and hence removing, carbon dioxide from the respiratory gases flowing through the interior of the cartridge.

The cartridge may be charged with material for treating respiratory gases by the end user, or more preferably the cartridge is supplied as a disposable unit that is already charged with such material. In either case, the end user preferably incorporates the charged cartridge into a respiratory circuit using attachment means that are well known in the art. The respiratory circuit will typically supply exhaled respiratory gases to the inlet of the cartridge, and remove treated respiratory gases from the outlet of the cartridge.

The inlet and outlet are preferably provided at opposite ends of the cartridge, and typically the Inlet is provided in the base of the cartridge. Material for treating respiratory gases, such as chemical absorbents for absorbing carbon dioxide, are generally granular in form. The inlet and/or outlet therefore preferably comprise a plurality of openings formed in a wall of the cartridge, the openings being sufficiently small to retain the granular material within the cartridge. Most preferably, the inlet and outlet each have the form of a mesh.

In preferred embodiments, the cartridge comprises a generally cylindrical container with a base and an open upper end, and a lid that is releasably engaged with the open upper end of the container.

The annular nature of the inlet reduces the proportion of the respiratory gas that flows directly into a central region of the cartridge.

The respiratory gases are preferably guided transversely relative to the direction of flow through the interior of the cartridge before there is any interaction between the respiratory gases and the material within the cartridge. In particular, the cartridge preferably defines one or more paths of least resistance for the respiratory gases that each extend from an opening of the inlet and each extend transversely relative to the surface of the material within the cartridge that is adjacent to the inlet, such that respiratory gases flow along a path of least resistance before flowing through the material within the cartridge.

In presently preferred embodiments, the cartridge comprises a plurality of formations that define the plurality of channels on the interior surface of the cartridge along which respiratory gases flow before interacting with the material within the cartridge. The channels are preferably arranged across the entire end of the cartridge in which the inlet Is formed.

Most preferably, each channel includes an open face, and the material within the cartridge contacts the formations so as to cover those open faces but not to pass between the formations into the channels. The path of least resistance for respiratory gases flowing through the openings of the inlet will therefore be along the channels rather than through the material within the cartridge. However, once the channels are charged along their full extent, the respiratory gases will flow through the material. Hence, the respiratory gas will flow substantially uniformly throughout the interior of the cartridge. Where the inlet is formed in the base of the cartridge, the material within the cartridge will be supported by the upper surface of the formations defining the channels.

In preferred embodiments, the formations have the form of ribs that are each orientated parallel to an adjacent rib so as to define a channel therebetween. Such ribs may be generally linear in form, and may be arranged in sets of similarly orientated ribs. In any case, the ribs are preferably arranged such that the channels defined by the ribs cause the respiratory gases to flow uniformly over the end of the cartridge in which the inlet is formed before interacting with the material within the cartridge.

According to a further aspect of the invention, there is provided a respiratory circuit incorporating a cartridge as hereinbefore described. Typically, the respiratory circuit will be a respiratory anaesthesia circuit.

The invention will now be described in greater detail, by way of illustration only, with reference to the accompanying drawings, in which
Figure 1 is a side view of a prior art cartridge comprising a container and lid;
Figure 2 is a plan view of the lid of the prior art cartridge;
Figure 3 is a view of the container of the prior art cartridge from above, with the lid of the cartridge removed;
Figure 4 is a perspective view, from above, of the container of the prior art cartridge;
Figure 5 is a plan view of a container forming part of a cartridge according to the invention;
Figure 6 is an underside view of the container of the cartridge according to the invention;
Figure 7 is a perspective view, from above, of the container of the cartridge according to the invention; and
Figure 8 is a perspective view of the container of the cartridge according to the invention in an inverted condition.

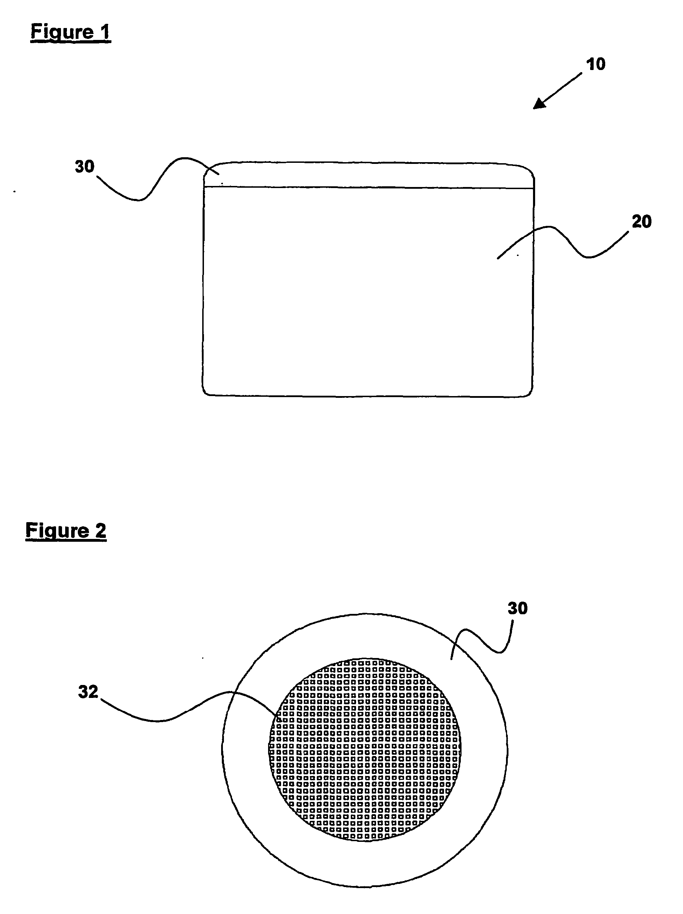

Figure 1 shows a prior art cartridge 10 that is adapted to contain a material for treating respiratory gases. In use, the prior art cartridge 10 is incorporated into a respiratory circuit such that respiratory gases flow through the interior of the cartridge 10 and are treated by the material contained therewithin.

Conventionally, the prior art cartridge 10 contains a chemical absorbent (not shown in the Figures) for absorbing carbon dioxide from the respiratory gases flowing through the interior of the cartridge 10. A variety of different forms of such chemical absorbents are well known in the art. However, the chemical absorbent is usually granular in form with a minimum dimension in any axis of about 3mm or greater. In addition, the chemical absorbent normally includes a pH indicator, such as ethyl violet, that changes colour, eg from colourless to violet in the case of ethyl violet, when the chemical absorbent is exhausted and hence no longer able effectively to absorb carbon dioxide. Most chemical absorbents also produce heat and water on reaction with the respiratory gases, and so act to humidify and heat the respiratory gases flowing through the cartridge 10.

The prior art cartridge 10 shown in Figure 1 comprises a container 20 and a lid 30 which together house the chemical absorbent, in use, and allow the throughflow of respiratory gases. The container 20 is cylindrical in form with a generally planar base and an open upper end (as viewed in Figure 1). The lid 30 is circular and includes a downwardly extending skirt at its periphery. The downwardly extending skirt includes a lower portion that is received within the upper end of the container 20 with a close, interference fit, and an upper portion having a greater external diameter that abuts the upper edge of the container 20. In this way, the lid 30 is releasably engaged with the container 20.

The container 20 and lid 30 are each formed in plastics material as a unitary component. At least the side wall of the container 20, and conventionally the entire prior art cartridge 10, is sufficiently translucent for any colour change of the chemical absorbent within the cartridge 10 to be readily visible.

As shown in Figure 2, the lid 30 includes a circular mesh 32 comprising a regular array of square openings. The circular mesh 32 is situated at the centre of the lid 30, and extends radially a distance of about half of the radius of the lid 30. An annular portion of the lid 30, which is continuous in form, surrounds the mesh 32. The openings of the mesh 32 allow respiratory gases to flow out of the prior art cartridge 10 through that part of the lid 30.

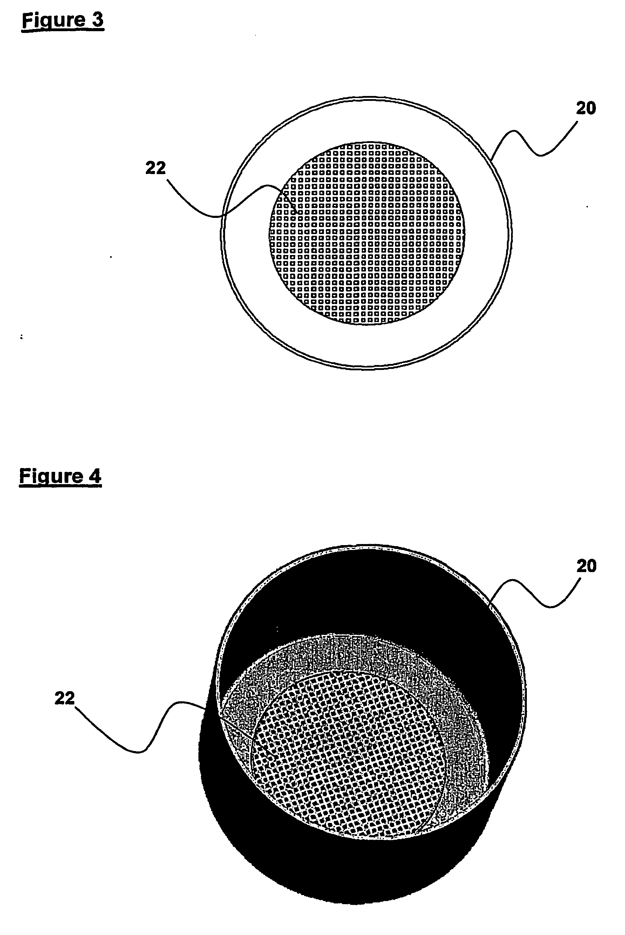

Figures 3 and 4 show the container 20 of the prior art cartridge 10, and illustrate that the base of the container 20 includes a circular mesh 22 that Is identical in form to the circular mesh 32 of the lid 30. An annular portion of the base of the container 20, which is continuous in form, surrounds the mesh 22. The openings of the mesh 22 allow respiratory gases to flow into the prior art cartridge 10 through that part of the base of the container 20.

The external surface of the base of the container 20, and the external surface of the lid 30, are both adapted to engage respiratory apparatus such that exhaled respiratory gases are supplied to the prior art cartridge 10 through the mesh 22 of the container 20 and treated respiratory gases are discharged through the mesh 32 of the lid 30. Such engagement means are well known in the art, and typically take the form of formations that are integrally formed with the container 20 and lid 30.

In use, the prior art cartridge 10 is charged with a suitable granular chemical absorbent, as discussed above. The chemical absorbent will have minimum granular dimensions such that granules of the absorbent cannot pass through the mesh 22,32 of the container 20 or lid 30. Conventionally, the prior art cartridge 10 is orientated in an upright position during use, as shown in Figure 1, and is charged with a chemical absorbent up to a level that is approximately 5mm below the interior surface of the lid 30. The cartridge 10 is incorporated into a respiratory circuit by means that are well known in the art.

Respiratory gases flow through the prior art cartridge 10 and react with the chemical absorbent such that carbon dioxide is removed from the respiratory gases and absorbed by the chemical absorbent. When the chemical absorbent is exhausted, and hence can no longer efficiently absorb carbon dioxide, the pH indicator in the chemical absorbent will change colour, eg from colourless to violet or from pink to white, so as to indicate to the user that the chemical absorbent is exhausted.

The prior art cartridge 10 described above with reference to Figures 1 to 4 suffers from the major disadvantage that respiratory gases tend to flow predominantly through a central cylindrical channel of the interior of the cartridge 10 and hence a central cylindrical core of the chemical absorbent. This means that a central cylindrical core of the chemical absorbent will become exhausted before the surrounding chemical absorbent, and hence there is non-uniform use of the chemical absorbent within the cartridge 10. As a consequence, the cartridge 10 will be become ineffective, and will need to be replaced, before an outer portion of the chemical absorbent has been fully exhausted. This is clearly a waste of the chemical absorbent. In addition, any colour change of the chemical absorbent in the central core will not be visible through the side wall of the container, and hence a user is given no indication of the inactivity of the cartridge until a carbon dioxide warning indicator, which is conventionally included in a respiratory circuit, is activated.

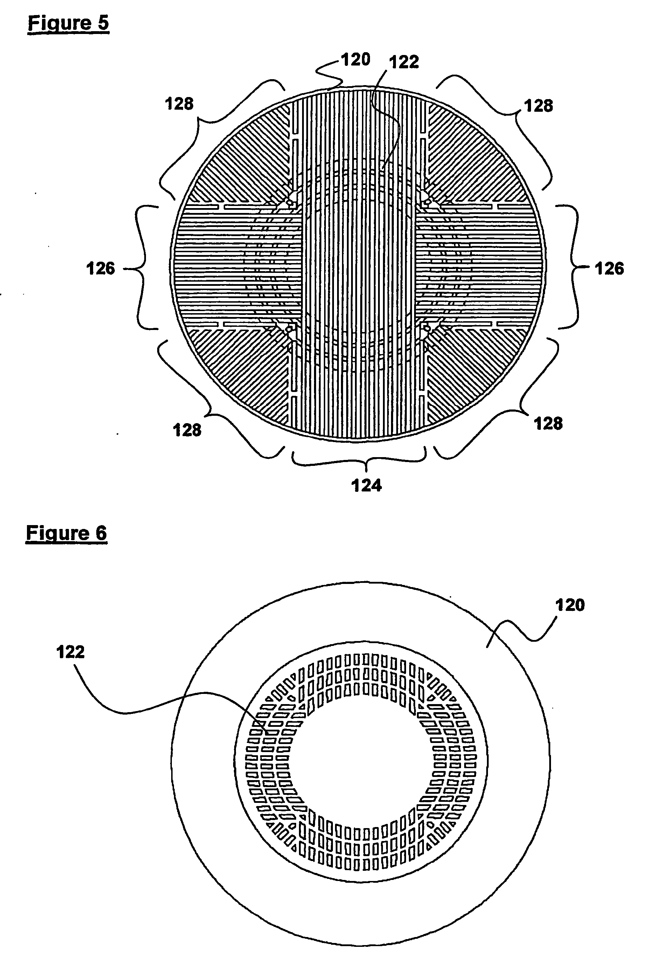

An example of a cartridge according to the invention comprises a container 120 as shown in Figures 5 to 8 and a lid (not shown in Figures 5 to 8) that is identical to the lid 30 of the prior art cartridge 10 described above. The cartridge according to the invention differs from the prior art cartridge 10 only In relation to the form of the container 120 of the cartridge, as described below.

The container 120 shown in Figures 5 to 8 is cylindrical in form with a generally planar base and an open upper end that engages with the lid. The container 120 is injection moulded in plastics material as a unitary component.

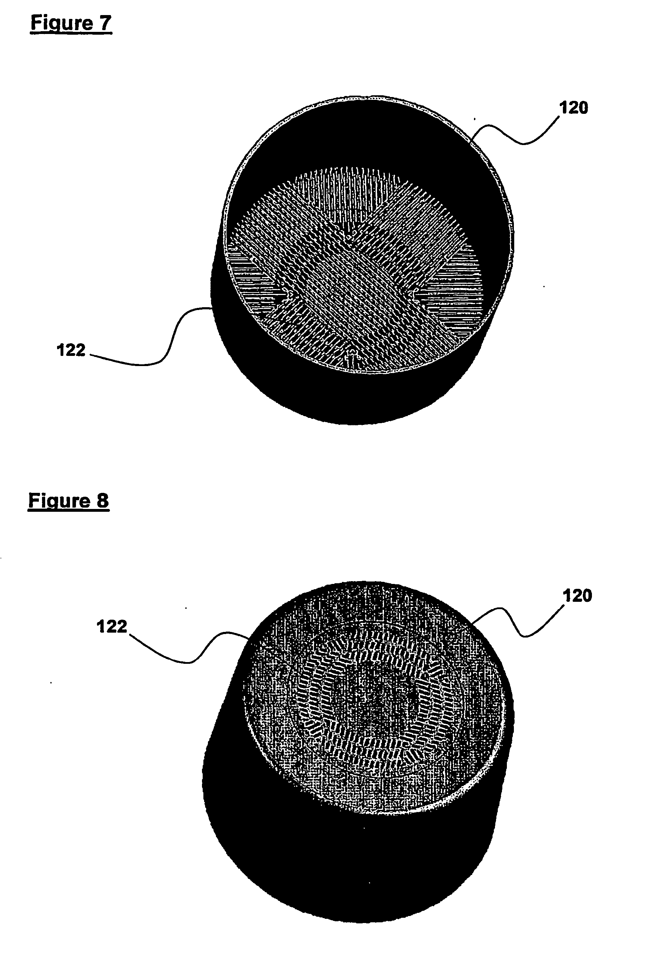

The exterior surface of the base of the container 120, as shown in Figures 6 and 8, includes an annular mesh 122 of openings, the majority of which have the general shape of a rectangle or parallelogram. The mesh 122 is surrounded by a continuous annular portion of the base of the container 120, and the mesh 122, in turn, surrounds a continuous circular disc at the centre of the base of the container 120. The openings of the mesh 122 enable respiratory gases to flow into the cartridge through that part of the base of the container 120.

The interior surface of the base of the container 120, as shown in Figures 5 and 7, includes a plurality of ribs 124,126,128 that are arranged across the entire interior surface of the base. The ribs 124,126,128 are each generally linear in form and extend between the openings of the mesh 122. In this arrangement, the ribs 124,126,128 cooperate to define a plurality of channels through which respiratory gases flow, in use. In particular, each opening of the mesh 122 opens into one of the channels formed by the ribs 124,126,128.

The ribs 124,126,128 are arranged in a number of sets, with the ribs 124,126,128 of each set being oriented parallel to one another and each rib 124,126,128 being separated from an adjacent rib 124,126,128 so as to define a channel therebetween.

A first set of ribs 124 is provided that forms a band extending across a diameter of the base. Two second sets of ribs 126 are provided that are each orientated perpendicularly to the first set of ribs 124, and each forms a band that extends between the edge of the first set of ribs 124 and the periphery of the base. Finally, four third sets of ribs 128, which are each oriented generally radially and at 45° to both the first and second sets of ribs 124,126, extend over the remaining areas of the base. The inner end of each of the third ribs 128 is separated from the adjacent first or second rib 124,126 so that the ends of the channels defined by the third ribs 128 are connected and hence in communication. In this way, each channel defined by the third ribs 128 is in communication with an opening of the mesh 122. In addition, the first and second ribs 124,126 that are immediately adjacent to the ends of the third ribs 128 include discontinuities that allow the flow of respiratory gas therethrough, and hence further facilitate the flow of respiratory gas into the channels defined by the third ribs 128.

In use, the cartridge is charged with a suitable granular chemical absorbent up to a level that is approximately 5mm below the interior surface of the lid. The chemical absorbent will have minimum granular dimensions such that granules of the absorbent cannot pass between adjacent ribs 124,126,128 into the channels defined by the ribs 124,126,128, and hence cannot pass through the mesh 122 of the container 120. When the cartridge has been charged with the chemical absorbent, a body of chemical absorbent will therefore rest upon, and be supported by, the upper surfaces of the ribs 124,126,128 such that the respiratory gases are free to flow unimpeded along the channels defined by the ribs 124,126,128.

The cartridge may be charged by the end user, or more preferably the cartridge is supplied as a disposable unit that is already charged. In either case, the end user incorporates the charged cartridge into a respiratory circuit by means that are well known in the art.

Exhaled respiratory gases are supplied to the openings of the mesh 122, and flow therethrough. These respiratory gases will then follow a path of least resistance, and hence flow along the channels that are defined by the ribs 124,126,128 and extend from the openings of the mesh 122. Respiratory gases will then flow upwardly from all points of each channel into and through the body of chemical absorbent. Since the channels defined by the ribs 124,126,128 offer a path of least resistance, the channels will remain charged with respiratory gases and respiratory gases will continue to flow upwardly from all points of each channel during use.

Respiratory gases flowing through the body of chemical absorbent will react with the chemical absorbent such that carbon dioxide is removed from the respiratory gases and absorbed by the chemical absorbent. The ribs 124,126,128 and channels defined therebetween ensure that respiratory gases flow at the same rate through all parts of the body of chemical absorbent, thereby ensuring uniform usage of the chemical absorbent throughout the cartridge.

When the chemical absorbent is exhausted, and hence can no longer efficiently absorb carbon dioxide, the pH indicator in the chemical absorbent will change colour, eg from colourless to violet or from pink to white, so as to indicate to the user that the chemical absorbent is exhausted. Since respiratory gases are flowing at the same rate through all parts of the body of chemical absorbent, the chemical absorbent adjacent to the transparent side wall of the container 120 will change colour when the chemical absorbent throughout the cartridge is exhausted and hence the cartridge needs to be replaced.

The cartridge according to the present invention therefore significantly reduces the amount of chemical absorbent that is wasted by ensuring that the chemical absorbent is uniformly active throughout the cartridge. This increases the lifetime of a given size of cartridge, and hence reduces cost. In addition, as soon as the cartridge has become ineffective due to exhaustion of the chemical absorbent, the colour change of the indicator within the chemical absorbent will be readily visible through the translucent side wall of the cartridge.

Finally, the external diameter of the mesh 122 of the container 120 is substantially similar to the external diameter of the mesh 22 of the prior art container 20. Hence, the cartridge of the invention is able to cooperate with the attachment means of existing anaesthetic machines and other respiratory apparatus.

## Claims

1. A cartridge for incorporation within a respiratory circuit, the cartridge being adapted to contain a material for treating respiratory gases and comprising an inlet and an outlet such that respiratory gases flow, in use, through the interior of the cartridge and interact with said material,
**characterised in that** the inlet comprises a plurality of openings arranged in an annulus about a continuous central part of a base of the cartridge, and said openings are in communication with a plurality of channels on an interior surface of the cartridge for guiding the respiratory gas transversely relative to its direction of flow through the interior of the cartridge.

2. A cartridge as claimed in Claim 1, wherein the cartridge is a disposable unit that is charged with the material for treating respiratory gases.

3. A cartridge as claimed in Claim 1 or Claim 2, wherein the material for treating respiratory gases is a chemical absorbent for absorbing, and hence removing, carbon dioxide from the respiratory gases flowing through the interior of the cartridge.

4. A cartridge as claimed in any preceding claim, wherein the inlet and outlet are provided at opposite ends of the cartridge.

5. A cartridge as claimed in any preceding claim, wherein the material for treating respiratory gases is granular in form, and the inlet and/or outlet comprises a plurality of openings formed in a wall of the cartridge, the openings being sufficiently small to retain the granular material within the cartridge.

6. A cartridge as claimed in Claim 5, wherein the inlet and outlet each have the form of a mesh (122).

7. A cartridge as claimed in any preceding claim, wherein the cartridge comprises a plurality of formations (124,126,128) that define the plurality of channels on the interior surface of the cartridge along which respiratory gases flow before interacting with the material within the cartridge.

8. A cartridge as claimed in Claim 7, wherein the channels are arranged across the entire end of the cartridge in which the inlet is formed.

9. A cartridge as claimed in Claim 7 or Claim 8, wherein each channel includes an open face, and the material within the cartridge contacts the formations (124,126,128) so as to cover those open faces but not to pass between the formations (124,126,128) into the channels.

10. A cartridge as claimed in Claim 9, wherein the inlet is formed in the base of the cartridge, and the material within the cartridge is supported by the upper surface of the formations (124,126,128) defining the channels.

11. A cartridge as claimed in any one of Claims 7 to 10, wherein the formations (124,126,128) have the form of ribs (124,126,128) that are each orientated parallel to an adjacent rib (124,126,128) so as to define a channel therebetween.

12. A cartridge as claimed in Claim 11, wherein the ribs (124,126,128) are arranged such that the channels defined by the ribs (124,126,128) cause the respiratory gases to flow uniformly over the end of the cartridge in which the inlet is formed before interacting with the material within the cartridge.

13. A cartridge as claimed in Claim 11 or Claim 12, wherein the ribs (124,126,128) are generally linear in form.

14. A cartridge as claimed in Claim 13, wherein the ribs (124,126,128) are arranged in sets of similarly orientated ribs (124,126,128).

## Patentansprüche

1. Kartusche zum Einbringen in einen Atemwegskreislauf, wobei die Kartusche ausgebildet ist, ein Material zur Behandlung von Atmungsgasen zu enthalten, und einen Einlass und einen Auslass aufweist, derart, dass Atmungsgase bei Verwendung durch das Innere der Kartusche strömen und mit dem besagten Material zusammenwirken,
**dadurch gekennzeichnet, dass** der Einlass eine Vielzahl von Öffnungen aufweist, die in einem Kreisring um einen durchgehenden zentralen Teil eines Bodens der Kartusche angeordnet sind, und die besagten Öffnungen mit einer Vielzahl von Kanälen auf einer Innenfläche der Kartusche in Verbindung stehen, um das Atmungsgas quer zu seiner Strömungsrichtung durch das Innere der Kartusche zu leiten.

2. Kartusche nach Anspruch 1, wobei die Kartusche eine Wegwerfeinheit ist, die mit dem Material zur Behandlung von Atmungsgasen beschickt wird.

3. Kartusche nach Anspruch 1 oder 2, wobei das Material zur Behandlung von Atmungsgasen ein chemisches Absorptionsmittel zur Absorption und somit zum Entzug von Kohlendioxid aus den Atmungsgasen, die durch das Innere der Kartusche strömen, ist.

4. Kartusche nach einem der vorhergehenden Ansprüche, wobei der Einlass und der Auslass an entgegengesetzten Enden der Kartusche bereitgestellt werden.

5. Kartusche nach einem der vorhergehenden Ansprüche, wobei das Material zur Behandlung von Atmungsgasen eine körnige Form aufweist, und wobei der Einlass und/oder der Auslass eine Vielzahl von Öffnungen aufweisen/aufweist, die in einer Wand der Kartusche ausgebildet sind, wobei die Öffnungen klein genug sind, um das körnige Material in der Kartusche zurückzuhalten.

6. Kartusche nach Anspruch 5, wobei der Einlass und der Auslass jeweils die Form eines Netzes (122) aufweisen.

7. Kartusche nach einem der vorhergehenden Ansprüche, wobei die Kartusche eine Vielzahl von Ausbildungen (124, 126, 128) aufweist, die die Vielzahl von Kanälen auf der Innenfläche der Kartusche, entlang derer Atmungsgase strömen, bevor sie mit dem Material in der Kartusche zusammenwirken, definieren.

8. Kartusche nach Anspruch 7, wobei die Kanäle über das gesamte Ende der Kartusche, in dem der Einlass ausgebildet ist, angeordnet sind.

9. Kartusche nach Anspruch 7 oder 8, wobei jeder Kanal eine offene Seite aufweist, und wobei das Material in der Kartusche mit den Ausbildungen (124, 126, 128) in Berührung steht, so dass es diese offenen Seiten bedeckt, dabei jedoch nicht zwischen den Ausbildungen (124, 126, 128) in die Kanäle dringt.

10. Kartusche nach Anspruch 9, wobei der Einlass im Boden der Kartusche ausgebildet ist, und wobei das Material in der Kartusche von der oberen Fläche der Ausbildungen (124, 126, 128), die die Kanäle definieren, getragen wird.

11. Kartusche nach einem der Ansprüche 7 bis 10, wobei die Ausbildungen (124, 126, 128) die Form von Rippen (124, 126, 128) aufweisen, die jeweils parallel zu einer benachbarten Rippe (124, 126, 128) ausgerichtet sind, so dass sie einen Kanal zwischen sich definieren.

12. Kartusche nach Anspruch 11, wobei die Rippen (124, 126, 128) derart angeordnet sind, dass die durch die Rippen (124, 126, 128) definierten Kanäle bewirken, dass die Atmungsgase gleichmäßig über das Ende der Kartusche, in dem der Einlass ausgebildet ist, strömen, bevor sie mit dem Material in der Kartusche zusammenwirken.

13. Kartusche nach Anspruch 11 oder 12, wobei die Rippen (124, 126, 128) generell eine geradlinige Form aufweisen.

14. Kartusche nach Anspruch 13, wobei die Rippen (124, 126, 128) in Zusammenstellungen von in gleicher Weise ausgerichteten Rippen (124, 126, 128) angeordnet sind.

## Revendications

1. Cartouche pour incorporation à l'intérieur d'un circuit respiratoire, la cartouche étant adaptée pour contenir un matériau pour traiter des gaz respiratoires et comprenant une entrée et une sortie de telle manière à ce les gaz respiratoires s'écoulent, lors de l'utilisation, à l'intérieur de la cartouche et interagissent avec ledit matériau,
**caractérisée en ce que** l'entrée comprend une pluralité d'ouvertures disposées en un espace annulaire autour d'une partie centrale continue d'une base de la cartouche, et lesdites ouvertures sont en communication avec une pluralité de canaux sur une surface intérieure de la cartouche pour guider le gaz respiratoire transversalement par rapport à sa direction d'écoulement à l'intérieur de la cartouche.

2. Cartouche telle que revendiquée à la Revendication 1, où la cartouche est une unité jetable qui est chargée avec le matériau pour traiter des gaz respiratoires.

3. Cartouche telle que revendiquée à la Revendication 1 ou Revendication 2, où le matériau pour traiter des gaz respiratoires est un absorbant chimique pour absorber, et donc éliminer, le dioxyde de carbone des gaz respiratoires s'écoulant à l'intérieur de la cartouche.

4. Cartouche telle que revendiquée dans une quelconque revendication précédente, où l'entrée et la sortie sont prévues à des extrémités opposées de la cartouche.

5. Cartouche telle que revendiquée dans une quelconque revendication précédente, où le matériau pour traiter des gaz respiratoires est sous forme granulaire, et l'entrée et/ou la sortie comprend une pluralité d'ouvertures formées dans une paroi de la cartouche, les ouvertures étant suffisamment petites pour retenir le matériau granulaire à l'intérieur de la cartouche.

6. Cartouche telle que revendiquée à la Revendication 5, où l'entrée et la sortie ont chacune la forme d'un maillage (122).

7. Cartouche telle que revendiquée dans une quelconque revendication précédente, où la cartouche comprend une pluralité de formations (124, 126, 128) qui définissent la pluralité de canaux sur la surface intérieure de la cartouche le long desquels des gaz respiratoires s'écoulent avant d'interagir avec le matériau à l'intérieur de la cartouche.

8. Cartouche telle que revendiquée à la Revendication 7, où les canaux sont disposés sur l'extrémité tout entière de la cartouche dans laquelle l'entrée est formée.

9. Cartouche telle que revendiquée à la Revendication 7 ou Revendication 8, où chaque canal inclut une face ouverte, et le matériau à l'intérieur de la cartouche entre en contact avec les formations (124, 126, 128) de manière à recouvrir ces faces ouvertes mais à ne pas passer entre les formations (124, 126, 128) dans les canaux.

10. Cartouche telle que revendiquée à la Revendication 9, où l'entrée est formée dans la base de la cartouche, et le matériau à l'intérieur de la cartouche est soutenu par la surface supérieure des formations (124, 126, 128) définissant les canaux.

11. Cartouche selon une quelconque des Revendications de 7 à 10, où les formations (124, 126, 128) ont la forme de côtes (124, 126, 128) qui sont chacune orientées en parallèle à une côte adjacente (124, 126, 128) de manière à définir un canal entre celles-ci.

12. Cartouche telle que revendiquée à la Revendication 11, où les côtes (124, 126, 128) sont disposées de telle manière que les canaux définis par les côtes (124, 126, 128) font que les gaz respiratoires s'écoulent uniformément au-dessus de l'extrémité de la cartouche dans laquelle l'entrée est formée avant d'interagir avec le matériau à l'intérieur de la cartouche.

13. Cartouche telle que revendiquée à la Revendication 11 ou Revendication 12, dans laquelle les côtes (124, 126, 128) sont généralement de forme linéaire.

14. Cartouche telle que revendiquée à la Revendication 13, où les côtes (124, 126, 128) sont disposées en ensembles de côtes orientées de manière similaire (124, 126, 128).
